# FASCICULE DE BREVET EUROPEEN

(11) **EP 1 940 354 B1**
(45) Date de publication et mention de la délivrance du brevet: **06.05.2015**
(21) Numéro de dépôt: 06808223.9
(22) Date de dépôt: 28.09.2006
(51) Int. Cl.: A61K 9/107

(54) **PREPARATION MEDICAMENTEUSE VISQUEUSE INJECTABLE COMPRENANT DE L'ETHANOL ET UN COMPOSE LIPOSOLUBLE OPAQUE AUX RAYONS X**
ABSOLUTEN ALKOHOL UND EINE RADIO-OPAKE VERBINDUNG ENTHALTENDE VISKÖSE UND INJIZIERBARE MEDIKAMENTÖSE ZUBEREITUNG
INJECTABLE VISCOUS MEDICINAL PREPARATION COMPRISING ETHANOL AND AN X-RAY OPAQUE FAT-SOLUBLE COMPOUND

(30) Priorité: 30.09.2005 FR 0509978
(43) Date de publication de la demande: 09.07.2008
(73) Titulaire: Theron, Jacques, 14123 Fleury sur Orne (FR); Dompmartin, Anne, 14000 Caen (FR); Labbe, Daniel, 14000 Caen (FR)
(72) Inventeur: Theron, Jacques, 14123 Fleury sur Orne (FR); Dompmartin, Anne, 14000 Caen (FR); Labbe, Daniel, 14000 Caen (FR)
(74) Mandataire: Catherine, Alain
(86) Numéro de dépôt international: PCT/FR2006/002213
(87) Numéro de publication internationale: WO 2007/036646

(56) Documents cités:
- WO-A-97/04813
- MASON K P ET AL: "Coagulation abnormalities in pediatric and adult patients after sclerotherapy or embolization of vascular anomalies." AJR. AMERICAN JOURNAL OF ROENTGENOLOGY DEC 2001, vol. 177, no. 6, décembre 2001 (2001-12), pages 1359-1363, XP002448951 ISSN: 0361-803X
- LEE I-H ET AL: "Stable paclitaxel formulations in oily contrast medium" JOURNAL OF CONTROLLED RELEASE, ELSEVIER, AMSTERDAM, NL, vol. 102, no. 2, 2 février 2005 (2005-02-02), pages 415-425, XP004710968 ISSN: 0168-3659
- GARIN ET AL: "Effect of Stabilized Iodized Oil Emulsion on Experimentally Induced Hepatocellular Carcinoma in Rats" JOURNAL OF VASCULAR AND INTERVENTIONAL RADIOLOGY, SOCIETY OF CARDIOVASCULAR AND INTERVENTIONAL RADIOLOGY, RESTON, US, vol. 16, no. 6, juin 2005 (2005-06), pages 841-848, XP022010945 ISSN: 1051-0443
- JAE HYUNG PARK ET AL: "TRANSCATHETER ARTERIAL EMBOLIZATION OF UNRESECTABLE RENAL CELL CARCINOMA WITH A MIXTURE OF ETHANOL AND IODIZED OIL" CARDIOVASCULAR AND INTERVENTIONAL RADIOLOGY, SPRINGER VERLAG, INC., NEW YORK, NY, US, vol. 17, no. 6, 1994, pages 323-327, XP008029836 ISSN: 0174-1551
- WESSELING M ET AL: "Drug release from beads coated with an aqueous colloidal ethylcellulose dispersion, Aquacoat(R), or an organic ethylcellulose solution" EUROPEAN JOURNAL OF PHARMACEUTICS AND BIOPHARMACEUTICS, ELSEVIER SCIENCE PUBLISHERS B.V., AMSTERDAM, NL, vol. 47, no. 1, janvier 1999 (1999-01), pages 33-38, XP009070951 ISSN: 0939-6411
- GOH JEFFREY SEOW KUANG ET AL: "Methylcellulose as a rectal contrast agent for MR imaging of rectal carcinoma." AJR. AMERICAN JOURNAL OF ROENTGENOLOGY. MAY 2002, vol. 178, no. 5, mai 2002 (2002-05), pages 1145-1146, XP002394487 ISSN: 0361-803X
- YU J ET AL: "Y-oxine-ethiodol, a potential radiopharmaceutical for the treatment of liver cancer" APPLIED RADIATION AND ISOTOPES, ELSEVIER, OXFORD, GB, vol. 58, no. 5, mai 2003 (2003-05), pages 567-573, XP004423007 ISSN: 0969-8043
- BRANS ET AL: "Clinical applications of newer radionuclide therapies" EUROPEAN JOURNAL OF CANCER, PERGAMON PRESS, OXFORD, GB, vol. 42, no. 8, mai 2006 (2006-05), pages 994-1003, XP005431187 ISSN: 0959-8049

## Description

La présente invention concerne une préparation médicamenteuse visqueuse injectable comprenant de l'éthanol et un composé liposoluble opaque aux rayons X. Elle s'applique plus particulièrement, mais non exclusivement, au traitement des malformations veineuses telles que les angiomes veineux.

De façon générale, les malformations veineuses sont caractérisées par des tuméfactions bleutées, molles, compressibles, gonflant en position déclive ou à l'effort.

Elles peuvent avoir un volume plus ou moins important et être situées dans des zones d'accès difficiles ou délabrantes pour lesquelles une chirurgie d'exérèse est parfois difficile ou impossible.

L'éthanol pur a été proposé pour les traiter par voie percutanée en réalisant une sclérose de la malformation. Son efficacité est incontestable mais son maniement présente un risqué non négligeable pour les raisons suivantes :
- le produit sclérosant est liquide et sa diffusion en dehors de la zone pathologique n'est que partiellement contrôlée par la vérification soigneuse de la bonne position de l'aiguille d'injection dans la malformation sur une série angiographique préalable,
- il n'est pas radio-opaque et les zones atteintes par le produit sclérosant ne sont contrôlables ni pendant ni après l'injection,
- il a été décrit des cas rares mais graves de toxicité cardiaque et générale liés directement à l'injection d'éthanol dans des malformations veineuses.

Pour améliorer la sûreté et l'efficacité de l'éthanol pur, il a été proposé d'utiliser un mélange d'éthanol et un composé pour donner une préparation visqueuse tel que l'éthylcellulose, la dextrine ou l'un de ses dérivés. Ce mélange forme un gel dont les avantages principaux sont au nombre de deux :
- Il s'agit d'un gel injectable par une aiguille dans la malformation veineuse et la diffusion de ce gel dans des zones non désirées est nettement moins importante qu'avec l'éthanol pur,
- le gel, du fait de son caractère physique, reste proche de la zone injectée et entraîne un effet sclérosant très augmenté. La quantité nécessaire pour obtenir le même résultat est très diminuée par rapport à l'éthanol pur réduisant son risque toxique général.

Ce gel présente cependant deux inconvénients significatifs :
- sa non radio-opacité,
- une floculation immédiate du mélange sclérosant (éthanol/éthylcellulose) au contact des substances de contraste hydrosolubles habituellement utilisées en radiologie vasculaire qui rend difficile la progression du gel dans l'aiguille d'injection et impossible dans un cathéter.

L'article « Coagulation abnormalities in pédiatrie and adult patients after sclerotherapy or embolization of vascular anomalies » AJR. AMERICAN JOURNAL OF ROENTGENOLOGY DEC 2001, vol. 177, no. 6, pages 1359-1363 décrit exclusivement les compositions sclérosantes ou embolisantes suivantes :
(i) une composition comprenant exclusivement de l'alcool absolu ;
(ii) une composition comprenant exclusivement du tetradécyl sulfate de sodium ;
(iii) une composition comprenant un mélange d'alcool absolu et de tetradécyl sulfate de sodium ;
(iv) une composition comprenant le mélange de n-butyl-2-cyanoacrylate, d'éthiodol et de poudre de tungsten ;
(v) une composition comprenant des particules de mousse d'alcool polyvinylique ; et
(vi) des micro-ressorts de platine.

L'article "Stable paclitaxel formulations in oily contrast medium". Journal of controlled release. Elsevier. Amsterdam, NL, vol. 102, no. 2, 2 février 2005 pages 415-425 concerne la résolution de problèmes de stabilité de formulations comprenant le principe actif paclitaxel et ne concerne donc aucunement les traitements d'embolisation.

L'article "Effect of Stabilized lodized Oil Emulsion on Experimentally Induced Hepatocellular Carcinoma in Rats" Journal of vascular and interventional radiology, Reston, US, vol. 16, no. 6 juin 2005 pages 841-848 a trait à la stabilisation d'émulsions de technetium radioactif dans du lipiodol, qui ne concerne aucunement les traitements par embolisation.

L'article « Transcatheter arterial embolization of unresectable rénal cell carcinoma with a mixture of ethanol and iodized oil » Cardiovascular and interventional radiology, Springer Verlag Inc., New York, NY, US, vol. 17, no. 6, 1994, pages 323-327 décrit des traitements d'embolisation artérielle avec des compositions comprenant un mélange d'éthanol et de lipiodol.

L'article « Drug release from beads coated with an aqueous colloidal ethylcellulose dispersion, Aquacoat(R), or an organic ethylcellulose solution" European Journal of Pharmaceutics and Biopharmaceutics, Elsevier Science Publishers B.V., Amsterdam, NL, vol. 47, no. 1, janvier 1999, pages 33-38 concerne une étude de la libération de principes actifs à partir de formes médicamenteuses solides comprenant un revêtement d'éthylcellulose. Ce document ne se situe aucunement dans le domaine technique de l'invention.

L'article « Methylcellulose as a rectal contrast agent for MR imaging of rectal carcinoma" American Journal of Roentgenology, May 2012, vol. 178, no.5, pages 1145-1146 décrit l'utilisation de methylcellulose comme agent de contraste pour des études d'imagerie endorectale et ne se situe donc pas dans le domaine de l'invention.

Le document WO 97/04813 A décrit des compositions embolisantes à trois composants comprenant (i) du diacétate de cellulose, (ii) un agent de contraste insoluble dans l'eau et (iii) un solvant biocompatible.

L'invention a pour objet de résoudre les inconvénients décrits ci-dessus.

A cet effet, elle propose une préparation médicamenteuse injectable comprenant de l'éthanol et au moins un composé liposoluble au moins partiellement opaque aux rayons X.

Selon l'invention, cette préparation est caractérisée en ce qu'elle comprend en outre un composé soluble dans l'éthanol qui présente en solution une viscosité comprise entre 10 et 700 cp et de préférence entre 90 et 350 cP à 25°C de manière à obtenir un gel à effet embolisant et, en ce que l'éthanol présente une pureté comprise entre 70 et 99% en volume, de préférence entre 90 et 99% de manière à obtenir un effet sclérosant.

Il est à noter que cP correspond à une unité de viscosité dynamique, le centipoise, qui équivaut à 10⁻³ pascal-seconde (Pa.s).

Bien entendu, ledit composé présentant en solution dans l'éthanol une viscosité comprise entre 10 et 700 cP et de préférence entre 90 et 350 cP à 25°C inclus au moins un composé liposoluble au moins partiellement opaque aux rayons X. Ces composés sont inertes les uns par rapport aux autres.

Des principes actifs pourront être incorporés dans la préparation selon l'invention.

Plus précisément, cette préparation pourra se composer d'une émulsion entre une phase visqueuse comprenant au moins l'éthanol et une phase liposoluble comprenant au moins ledit composé liposoluble au moins partiellement opaque aux rayons X.

Ladite préparation selon l'invention pourra comprendre de 10% à 50% en volume dudit composé liposoluble au moins partiellement opaque aux rayons X.

Avantageusement, plus l'émulsion sera composée de particules fines plus le mélange sera homogène et plus le suivi du trajet du mélange lors de l'injection sera bien rendu.

Ladite émulsion pourra être réalisée par incorporation dudit composé liposoluble au moins partiellement opaque aux rayons X soit à la fin de la fabrication de la préparation, soit juste avant l'intervention.

Le composé liposoluble au moins partiellement opaque aux rayons X pourra être un composé non-métallique.

Ce composé non-métallique pourra être un ester d'acide gras halogéné tel qu'un ester éthylique d'acide gras iodé. Avantageusement, dans le cas de malformations veineuses proches de la peau qui sont souvent traitées pour des raisons essentiellement esthétiques, un composé non-métallique pourra permettre d'éviter une coloration noire de la peau telle qu'elle aurait lieue en utilisant des composés métalliques.

Ledit composé présentant en solution une viscosité comprise entre 10 et 700 cp et de préférence entre 90 et 350 cP à 25°C pourra être choisi de façon à présenter les propriétés suivantes :
- biocompatible, en raison du caractère injectable de la préparation,
- un pouvoir épaississant suffisamment important pour augmenter, même présent en faible quantité, la viscosité du mélange,
- des dérivés biodegradables in situ afin de dispenser d'une résection chirurgicale de la zone soignée,
- une solubilité à froid dans l'éthanol de manière à obtenir une préparation homogène,
- des effets toxiques locaux et/ou systémiques réduits au minimum voire inexistants pour ne pas compromettre la tolérance de la préparation
et éventuellement une présentation sous forme de poudre et non de liquide afin de ne pas diluer l'éthanol.

Ledit composé présentant une viscosité comprise entre 10 et 700 cp et de préférence entre 90 et 350 cP à 25°C pourra être l'éthylcellulose.

L'éthylcellulose pourra être présent en une concentration variant de 0,5 à 30 % et de préférence de 5 à 18 % en poids du poids total de la préparation.

Par conséquent, la préparation selon l'invention répond aux critères essentiels requis qui sont la sûreté d'emploi dans toutes les phases du processus, l'aptitude à créer une sclérose stable et sélective.

Un mode d'exécution de l'invention sera décrit ci-après, à titre d'exemple non limitatif, avec référence aux dessins annexés dans lesquels :
La figure 1 représente la mesure de la viscosité exprimée en centipoise en fonction de la concentration exprimée en pourcentage du poids d'éthylcellulose par rapport au poids total ;
La figure 2 représente la mesure de la viscosité exprimée en centipoise en fonction de la température exprimée en degré Celsius.

### Fabrication de la préparation

Plusieurs préparations à différentes concentrations en éthylcellùlose (Aqualon 100 NF ®, Hercules) ont été effectuées par dissolution respectivement de 0,15 ; 0,45 et 0,75 g dans 15 mL d'éthanol d'une pureté de 70 à 99 % en volume et de préférence de 95 % (d = 0,8) soit 1,22 ; 3,61 et 5,88 % en poids du poids total de la préparation.

La préparation dont la concentration en éthylcellulose est la plus élevée a été choisie. Une perte de 2,5 % au moment de la répartition en flacons a été considéré soit pour quarante flacons, des proportions de 205 mL d'alcool à 95° (% volume) et 10,25 g d'éthylcellulose.

Plus généralement, l'éthylcellulose est présent en une concentration variant de 5 à 18 %, de préférence 5,88 % en poids du poids total de la préparation.

Conformément aux Bonnes Pratiques de Fabrication (1998), la préparation a été réalisée en trois étapes: préparation du gel, répartition aseptique et stérilisation du produit dans le conditionnement final. Dans un premier temps, l'excipient (éthylcellulose) a été mélangé à l'éthanol dans un ballon à col rodé stérile, à chaud, par agitation magnétique et à reflux jusqu'à dissolution complète. Le tout a été laissé sous agitation et avec le reflux pendant quinze minutes, puis sous agitation jusqu'à refroidissement afin de permettre la recondensation de l'alcool dans le ballon. Le reconditionnement s'est fait ensuite sous hotte à flux laminaire horizontal dans des flacons sertis stériles de 5 mL (Bioblock 42065). Enfin, comme le préconise la Pharmacopée européenne, les flacons ont été stérilisés à l'autoclave par vapeur saturée, à 121 °C pendant vingt minutes.

La dernière étape de fabrication de la préparation, l'ajout d'une substance opacifiante liposoluble tel qu'un ester éthylique d'acide gras iodé (Laboratoire Guerbet, France), se fait en proportion variable pour obtenir une bonne opacité, par exemple de l'ordre de 10% à 50% en volume du volume total.

Cette substance (phase liposoluble) n'étant pas miscible avec le mélange éthanol/éthylcellulose (phase visqueuse), le résultat de l'agitation des deux phases conduit à une émulsion dont la taille des particules est fonction de l'agitation appliquée.

Il est à noter que plus les particules sont fines, plus le mélange injecté sera homogène et plus la qualité du suivi de l'injection sera bonne.

Ce dernier ajout pourra se faire par exemple dans la seringue avant injection.

Par ailleurs, cette substance étant inerte et utilisée en faible quantité, elle ne modifie pas significativement les résultats des contrôles présentés ci-dessous et effectués sur la préparation avant leur ajout.

### Contrôles effectués

La conformité de la préparation a été vérifiée par un essai de stérilité, des contrôles chimiques et physicochimiques.

Selon les recommandations de la Pharmacopée européenne, la présence éventuelle de contaminants a été recherchée par ensemencement de 4 mL de la préparation dans 250 mL de bouillon tryticase-soja pour les germes aérobies, au thioglycolate pour les germes anaérobies et Sabouraud pour les levures.

Les résultats de l'essai de stérilité ont confirmé l'absence de contaminants dans la préparation.

La teneur en alcool a été déterminée après dilution de l'échantillon et incorporation de l'étalon interne, le propanol-1, par chromatographie gazeuse avec détection par ionisation de flamme. La séparation est obtenue sur une colonne Porapak Q (80-100 « mesh » (maille), longueur 3 m) avec l'azote comme gaz vecteur (1,2 bar) sur un appareil Delsi DN200.

Le dosage de l'alcool a donné une valeur de 802 g.L⁻¹.

Le dosage spécifique de l'adjuvant de viscosité n'a pas été réalisé, mais la concentration a été estimée par la méthode des résidus secs, opération qui consiste à évaporer l'éthanol dans une cuve thermostatée à 110°C jusqu'à poids constant de l'échantillon.

La méthode des résidus secs a permis de corréler la concentration théorique en éthylcellulose à celle mesurée expérimentalement soit 5,88 % en poids du poids total de l'échantillon.

La viscosité de la préparation a été mesurée à l'aide d'un viscosimètre à capillaire dit de Baumé (Prolabo). Plusieurs séries de mesure ont été effectuées à différentes températures et à différentes concentrations en épaississant la préparation.

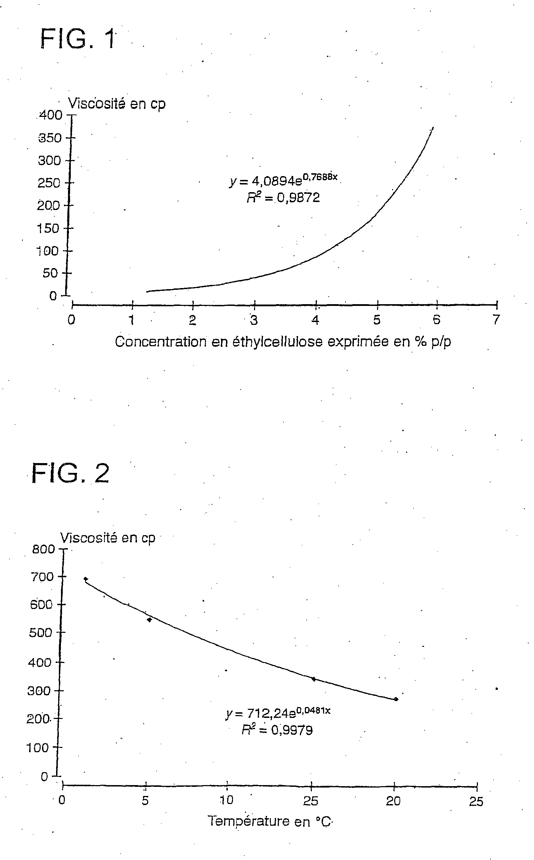

Les mesures de viscosité ont montré que, à température constante, la viscosité de la préparation augmentait de manière exponentielle avec la teneur en éthylcellulose (Figure 1). En revanche, elle diminuait, toujours de façon exponentielle, lorsque la température augmentait (Figure 2).

Enfin l'étude de la stabilité physicochimique a été réalisée par une analyse dans le temps de l'évolution des paramètres qui définissent la préparation c'est-à-dire la viscosité, la teneur en éthanol et en agent de viscosité. Les mesures ont été répétées à un jour (J1), huit jours (J8), quinze jours (J15), trente jours (J30).

Les résultats sont consignés dans le tableau I. Les coefficients de variation inférieurs à 3% prouvent la stabilité du mélange jusqu'à J30, ce qui permettra de déterminer une date de péremption pour la préparation.

**Tableau I : Paramètres physicochimiques en fonction du temps**

| Date | C_{éthanol} (g.L⁻¹) | V_{éch} (mL) | P_{éch} (g) | d_{éch} (g.mL^{- 1}) | Résidu sec RS (g) | Rapport RS/P_{éch} (%) | Viscosité (cp)* |
|---|---|---|---|---|---|---|---|
| J1 | 784 | 2 | 1,650 | 0,825 | 0,101 | 6,10 | 320 |
| J8 | 821 | 2 | 1,720 | 0,850 | 0,103 | 5,97 | 339,5 |
| J15 | 783 | 2 | 1,610 | 0,800 | 0,097 | 6,00 | - |
| J30 | 820 | 2 | 1,670 | 0,830 | 0,099 | 5,92 | 332 |
| Moyenne | 802 | - | 1,663 | 0,826 | 0,100 | 5,998 | 330,5 |
| Ecart-type | 21,370 | - | 0,046 | 0,021 | 0,002 | 0,076 | 9,836 |
| Coefficient de variation | 2,665 | - | 2,751 | 2,489 | 2,458 | 1,265 | 2,976 |

| | | | | | | | |
|---|---|---|---|---|---|---|---|
| * cp : centipoise | | | | | | | |

## Revendications

1. Préparation médicamenteuse injectable comprenant de l'éthanol et au moins un composé liposoluble au moins partiellement opaque aux rayons X, **caractérisée en ce qu'**elle comprend en outre un composé soluble dans l'éthanol qui présente en solution une viscosité comprise entre 10 et 700 cp et de préférence entre 90 et 350 cP à 25°C de manière à obtenir un gel à effet embolisant et, **en ce que** l'éthanol présente une pureté comprise entre 70 et 99% en volume, de préférence entre 90 et 99% de manière à obtenir un effet sclérosant.

2. Préparation selon la revendication 1,
**caractérisée en ce que** ledit composé présentant en solution une viscosité comprise entre 10 et 700 cp, l'éthanol et le au moins un composé liposoluble au moins partiellement opaque aux rayons X sont inertes les uns par rapport aux autres.

3. Préparation selon la revendication 1,
**caractérisée en ce qu'**elle se. compose d'une émulsion entre une phase visqueuse comprenant au moins l'éthanol et une phase liposoluble comprenant ledit composé liposoluble au moins partiellement opaque aux rayons X.

4. Préparation selon la revendication 1,
**caractérisée en ce qu'**elle comprend environ 10% à 50% en volume dudit composé liposoluble au moins partiellement opaque aux rayons X.

5. Préparation selon la revendication 1,
**caractérisée en ce que** le composé liposoluble au moins partiellement opaque aux rayons X est un composé non-métallique.

6. Préparation selon la revendication 5,
**caractérisée en ce que** ledit composé liposoluble au moins partiellement opaque aux rayons X est un ester d'acide gras halogéné.

7. Préparation selon la revendication 6,
**caractérisée en ce que** ledit ester d'acide gras halogéné est un ester éthylique d'acide gras iodé.

8. Préparation selon la revendication 1,
**caractérisée en ce que** les caractéristiques dudit composé présentant en solution une viscosité comprise entre 10 et 700 cp à 25°C sont les suivantes :
- biocompatible,
- un pouvoir épaississant suffisamment important pour augmenter, même présent en faible quantité, la viscosité du mélange,
- des dérivés biodégradables in situ,
- une solubilité à froid dans l'éthanol,
- des effets toxiques locaux et/ou systémiques réduits au minimum.

9. Préparation selon la revendication 1,
**caractérisée en ce que** ledit composé présentant en solution une viscosité comprise entre 10 et 700 cp à 25°C se présente sous forme de poudre.

10. Préparation selon l'une des revendications précédentes,
**caractérisée en ce que** ledit composé présentant en solution une viscosité comprise entre 10 et 700 cp à 25°C est l'éthylcellulose, la dextrine ou l'un de ses dérivés.

## Patentansprüche

1. Injizierbares Arzneipräparat, das Ethanol und wenigstens eine fettlösliche Verbindung, die wenigstens teilweise röntgenopak ist, umfasst, **dadurch gekennzeichnet, dass** es außerdem eine in Ethanol lösliche Verbindung umfasst, die in Lösung eine Viskosität bei 25 °C zwischen 10 und 700 cP und vorzugsweise zwischen 90 und 350 cP aufweist, so dass man ein Gel mit embolisierender Wirkung erhält, und dadurch, dass Ethanol eine Reinheit zwischen 70 und 99 Vol.-%, vorzugsweise zwischen 90 und 99 Vol.- %, aufweist, so dass man eine sklerotisierende Wirkung erhält.

2. Präparat gemäß Anspruch 1, **dadurch gekennzeichnet, dass** die Verbindung, die in Lösung eine Viskosität zwischen 10 und 700 cP aufweist, das Ethanol und die wenigstens eine fettlösliche Verbindung, die wenigstens teilweise röntgenopak ist, gegeneinander inert sind.

3. Präparat gemäß Anspruch 1, **dadurch gekennzeichnet, dass** es sich aus einer Emulsion zwischen einer viskosen Phase, die wenigstens das Ethanol umfasst, und einer fettlöslichen Phase, die die fettlösliche Verbindung, die wenigstens teilweise röntgenopak ist, umfasst, zusammensetzt.

4. Präparat gemäß Anspruch 1, **dadurch gekennzeichnet, dass** es ungefähr 10 bis 50 Vol.-% der fettlöslichen Verbindung, die wenigstens teilweise röntgenopak ist, umfasst.

5. Präparat gemäß Anspruch 1, **dadurch gekennzeichnet, dass** die fettlösliche Verbindung, die wenigstens teilweise röntgenopak ist, eine Nichtmetallverbindung ist.

6. Präparat gemäß Anspruch 5, **dadurch gekennzeichnet, dass** die fettlösliche Verbindung, die wenigstens teilweise röntgenopak ist, ein halogenierter Fettsäureester ist.

7. Präparat gemäß Anspruch 6, **dadurch gekennzeichnet, dass** der halogenierte Fettsäureester ein iodierter Fettsäureethylester ist.

8. Präparat gemäß Anspruch 1, **dadurch gekennzeichnet, dass** die Merkmale der Verbindung, die in Lösung eine Viskosität bei 25 °C zwischen 10 und 700 cP aufweist, die folgenden sind:
- biokompatibel;
- eine ausreichend hohe Verdickungsfähigkeit, um selbst in geringer Menge die Viskosität des Gemischs zu erhöhen;
- in situ biologisch abbaubare Derivate;
- Löslichkeit in kaltem Ethanol;
- auf ein Minimum reduzierte lokale und/oder systemische toxische Wirkungen.

9. Präparat gemäß Anspruch 1, **dadurch gekennzeichnet, dass** die Verbindung, die in Lösung eine Viskosität bei 25 °C zwischen 10 und 700 cP aufweist, in Form eines Pulvers vorliegt.

10. Präparat gemäß einem der vorstehenden Ansprüche, **dadurch gekennzeichnet, dass** es sich bei der Verbindung, die in Lösung eine Viskosität bei 25 °C zwischen 10 und 700 cP aufweist, um Ethylcellulose, Dextrin oder eines seiner Derivate handelt.

## Claims

1. An injectable medicinal preparation comprising ethanol and at least one at least partially X-ray opaque, fat-soluble compound, **characterized in that** it further comprises an ethanol-soluble compound which, when dissolved, has a viscosity ranging from 10 to 700 cP and preferably from 90 to 350 cP at 25°C so as to provide an embolizing effect-inducing gel, and, **in that** the ethanol has a purity ranging from 70 to 99% vol., preferably from 90 to 99% vol., so as to provide a sclerosing effect.

2. A preparation according to claim 1, **characterized in that** said compound having, when dissolved, a viscosity ranging from 10 to 700 cP, the ethanol and the at least one at least partially X-ray opaque, fat-soluble compound are inert toward each other.

3. A preparation according to claim 1, **characterized in that** it is composed of an emulsion based on a viscous phase comprising at least ethanol and a fat-soluble phase comprising said at least partially X-ray opaque, fat-soluble compound.

4. A preparation according to claim 1, **characterized in that** it comprises from about 10% to 50% vol. of said at least partially X-ray opaque, fat-soluble compound.

5. A preparation according to claim 1, **characterized in that** the at least partially X-ray opaque, fat-soluble compound is a non metallic compound.

6. A preparation according to claim 5, **characterized in that** said at least partially X-ray opaque, fat-soluble compound is a halogenated fatty acid ester.

7. A preparation according to claim 6, **characterized in that** said halogenated fatty acid ester is an iodine fatty acid ethyl ester.

8. A preparation according to claim 1, **characterized in that** said compound having, when dissolved, a viscosity ranging from 10 to 700 cP at 25°C has the following characteristics:
- it is biocompatible,
- it has a thickening power sufficient to increase the mixture viscosity, even in a small amount,
- it comprises *in situ* biodegradable derivatives,
- it is soluble in cold ethanol,
- it has maximally reduced local and/or systemic toxic effects.

9. A preparation according to claim 1, **characterized in that** said compound having, when dissolved, a viscosity ranging from 10 to 700 cP at 25°C comes as a powder.

10. A preparation according to any preceding claim, **characterized in that** said compound having, when dissolved, a viscosity ranging from 10 to 700 cP at 25°C is ethylcellulose, dextrin or a derivative thereof.
